# EUROPEAN PATENT SPECIFICATION

(11) **EP 4 355 421 B1**
(45) Date of publication and mention of the grant of the patent: **16.09.2026**
(21) Application number: 22825773.9
(22) Date of filing: 15.06.2022
(51) Int. Cl.: A61N 7/00, A61N 7/02, A61B 8/08, A61B 8/00, A61B 18/04, A61B 18/00

(54) **DEVICE FOR PAIN SUPPRESSION THROUGH TARGETED PERIPHERAL NERVE APPLICATION OF FOCUSED ULTRASOUND**
VORRICHTUNG ZUR SCHMERZUNTERDRÜCKUNG DURCH GEZIELTE PERIPHERE NERVENANWENDUNG VON FOKUSSIERTEM ULTRASCHALL
DISPOSITIF DE SUPPRESSION DE LA DOULEUR PAR APPLICATION CIBLÉE D'ULTRASONS FOCALISÉS SUR LES NERFS PÉRIPHÉRIQUES

(30) Priority: 15.06.2021 US 202163210864 P; 25.02.2022 US 202263314143 P
(43) Date of publication of application: 24.04.2024
(73) Proprietor: The Board of Trustees of the Leland Stanford Junior University, Stanford, CA 94305 (US)
(72) Inventor: ANDERSON, Thomas Anthony, Menlo Park, CA 94305 (US)
(74) Representative: Boult Wade Tennant LLP
(86) International application number: PCT/US2022/033687
(87) International publication number: WO 2022/266261

(56) References cited:
- KR-A- 20140 113 674
- KR-A- 20150 029 036
- US-A1- 2005 240 126
- US-A1- 2010 234 728
- US-A1- 2014 039 279
- US-A1- 2014 058 292
- US-A1- 2020 147 415
- US-A1- 2020 147 415

## Description

### TECHNICAL FIELD

The present application is related to devices, systems, and methods for delivering focused ultrasound into a subject's body and, more particularly, to devices, systems, and methods for relieving acute or chronic pain using focused ultrasound targeting one or more nerves of a subject having pain, e.g., with or without causing neuromodulation or ablation of the nerves.

### BACKGROUND

Subjects may experience peripheral nerve pain due to various circumstances. For example, a patient may experience acute pain, e.g., due to surgery or an injury, or the pain may be chronic pain, e.g., due to conditions such as complex regional pain syndrome (CRPS) type 1 and 2, phantom limb pain, trigeminal neuralgia, Bell's palsy, intercostal pain, post-herpatic neuralgia, endometriosis, and neuroma.

The most common method for pain management is narcotics, which may have a range of adverse side effects.

In US 2020/147415 A1, there are described systems for selective modulation of motor neuronal activity in a peripheral nervous system using a focused ultrasound assembly.

In US 2014/039279 A1, there are described methods and systems for identifying and spatially localizing tissue having certain physiological properties or producing certain biological responses. Targeted acoustic probing may be employed to identify the scope and severity of chronically painful sensitized tissue areas.

In US 2005/240126 A1, there is described a method for using high intensity focused ultrasound (HIFU) to treat neurological structures to achieve a desired therapeutic affect. For example, a relatively high dose of HIFU can be used to permanently block nerve function, to provide a non-invasive alternative to severing a nerve to treat sever spasticity.

Accordingly, devices and methods for improving pain management, e.g., for non-drug-based pain suppression, would be useful.

### SUMMARY

Various aspects and embodiments of the invention are set out in the appended claims.

The present application is related to devices, systems, and methods for delivering focused ultrasound into a subject's body. More particularly, devices, systems, and methods are provided for relieving pain using focused ultrasound to target one or more nerves of a subject having pain. In one example, focused ultrasound may be delivered to relieve pain without damaging the target nerve or other tissues. Alternatively, the focused ultrasound may be delivered to cause neuromodulation or ablation of the targeted nerve(s). The pain being treated may be acute pain, with indications including but not limited to acute surgical pain in which the pain is resolved through specific peripheral nerve blockade. The pain may otherwise be chronic pain, with indications including but not limited to complex regional pain syndrome (CRPS) type 1 and 2, phantom limb pain, trigeminal neuralgia, Bell's palsy, intercostal pain, post-herpatic neuralgia, endometriosis, and neuroma.

The use of focused ultrasound for neuromodulation has demonstrated success in a wide range of applications, from the removal of amyloid plaques in Alzheimer's to the disruption of electrographic seizure activity. Most prior work with low-intensity focused ultrasound has been used for transcranial excitation or inhibition of neural structures, such as direct activation of brain regions using transcranial focused ultrasound pulses or induced motor activity in response to transcranial focused ultrasound. High intensity ultrasound has been previously used for ablation of tissues, such as solid tumors. There has been limited prior work using low-intensity ultrasound for peripheral nervous system neuromodulation, such as partial vagus nerve inhibition.

However, there currently exists no device or procedure to use focused ultrasound for peripheral nervous system neuromodulation, including one or both of suppression and excitation of peripheral nerves, for the purposes of acute and chronic pain suppression. The majority of pain management is currently accomplished through narcotics, which have a range of side effects. Focused ultrasound may be used as an alternative, noninvasive and non-drug-based method for pain suppression.

In accordance with one example, a device is provided for relieving pain in a subject according to claim 1.

In one example, the devices herein may include one or more acoustic transducers coupled to a controller such that the device is configured to transmit low-intensity ultrasound, e.g., with an intensity up to five hundred Watts per square centimeter (i.e., between 0-500 W/cm²), e.g., at frequencies between about one kilohertz and ten megahertz (1 KHz-10 MHz). In another example, the devices may be configured to transmit high-intensity ultrasound, e.g., with an intensity between about five hundred and two thousand Watts per square centimeter (500-2000 W/cm²), e.g., at frequencies between about one kilohertz and ten megahertz (1 KHz-10 MHz).

In some examples, the ultrasound energy is emitted by the ultrasound transducer(s) substantially continuously. In other examples, the ultrasound energy is emitted intermittently. In yet other examples, the ultrasound energy is emitted in pulsatile form.

Optionally, the devices herein may be configured to deliver high-intensity ultrasound to cause thermal ablation or neurolysis of target nerves for pain reduction. Alternatively, the devices may utilize focused ultrasound on peripheral nerve tissue for acute and chronic pain reduction through both thermal and non-thermal mechanisms (i.e., high-intensity versus low-intensity ultrasound). Optionally, neuromodulation may be achieved through either or both excitation and suppression of nerve conduction or ultrasound energy transfer to tissue.

Other aspects and features of the present invention will become apparent from consideration of the following description taken in conjunction with the accompanying drawings.

### BRIEF DESCRIPTION OF THE DRAWINGS

It is believed the present invention will be better understood from the following description of certain examples taken in conjunction with the accompanying drawings, in which like reference numerals identify the same elements and in which:
FIG. 1 shows an example of a device for delivering focused ultrasound into a subject's body to a target nerve to suppress pain.
FIG. 2 is a schematic showing exemplary components that may be included in the device of FIG. 1.
FIG. 3 shows an alternative example of a device including an adjustable ultrasound imaging element.

The drawings are not intended to be limiting in any way, and it is contemplated that various examples of the invention may be carried out in a variety of other ways, including those not necessarily depicted in the drawings. The accompanying drawings incorporated in and forming a part of the specification illustrate several aspects of the present invention, and together with the description serve to explain the principles of the invention; it being understood, however, that this invention is not limited to the precise arrangements shown.

### DETAILED DESCRIPTION

The following description of certain examples of the invention should not be used to limit the scope of the present invention. Other examples, features, aspects, embodiments, and advantages of the invention will become apparent to those skilled in the art from the following description, which is by way of illustration, one of the best modes contemplated for carrying out the invention. As will be realized, the invention is capable of other different and obvious aspects, all without departing from the invention. Accordingly, the drawings and descriptions should be regarded as illustrative in nature and not restrictive.

Before the examples are described, it is to be understood that the invention is not limited to particular examples described, as such may, of course, vary. It is also to be understood that the terminology used herein is for the purpose of describing particular examples only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, some potential and exemplary methods and materials are now described.

It must be noted that as used herein and in the appended claims, the singular forms "a," "an," and "the" include plural referents unless the context clearly dictates otherwise. Thus, for example, reference to "a compound" includes a plurality of such compounds and reference to "the polymer" includes reference to one or more polymers and equivalents thereof known to those skilled in the art, and so forth.

Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number which, in the context in which it is presented, provides the substantial equivalent of the specifically recited number.

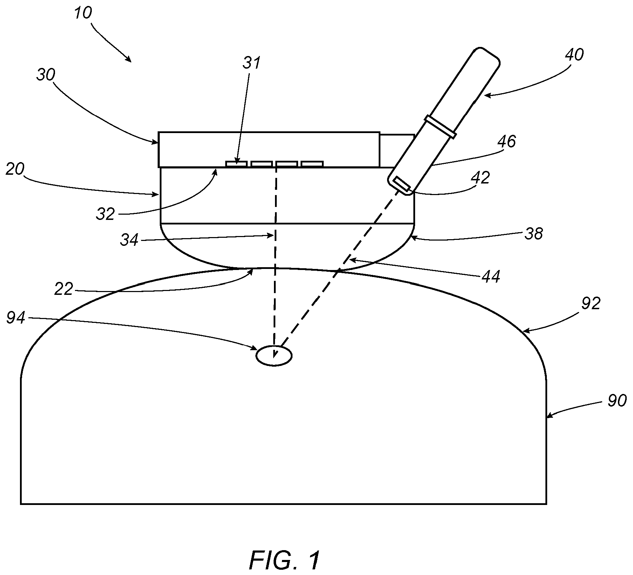

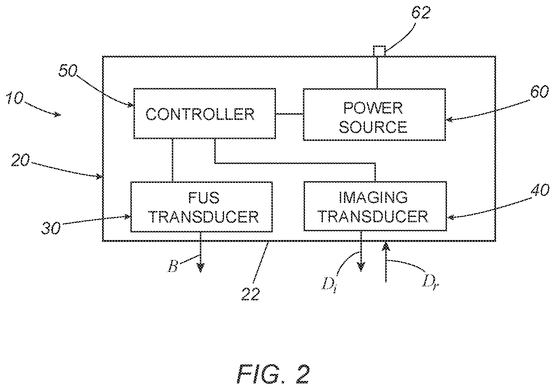

Turning to the drawings, FIG. 1 shows an example of a device 10 for relieving peripheral nerve pain in a subject's body 90 that includes a housing 20 containing one or more components for operating the device 10, e.g., a FUS transducer 30, an imaging or locating element 40, a controller 50, and a power source 60, as shown in FIG. 2. The housing 20 may include a contact surface 22 configured for placement against the subject's skin 92, e.g., to allow focused ultrasound to be delivered from transducer 30 through the surface 22 into the subject's body 90, as described further elsewhere herein.

Optionally, the housing 20 may be shaped to provide a handheld device, e.g., having a shape to facilitate the subject holding the device 10. For example, the housing 20 itself may be shaped to allow a user to hold the device 10, e.g., having an elongated cylindrical or other shape, and/or one or more handles, grips, or other features (not shown) may extend from the housing 20 to facilitate manipulation of the device 10 during use.

In addition or alternatively, the housing 20 may include one or more features to facilitate securing the device 10 relative to the subject's body 90. For example, the contact surface 22 may include adhesive or other tacky material that may secure the surface 22 to the skin 92 during use, but allow the device 10 to be removed without damaging the skin 92. In addition or alternatively, one or more straps (not shown) may be provided on the housing 20 that may be wrapped at least partially around the subject's body, e.g., a pair of straps on opposite sides of the contact surface 22 having sufficient length to allow the straps to be wrapped around an appendage, e.g., arm or leg, or the torso of the subject's body during use. Optionally, the straps may include ends with cooperating fasteners, e.g., hook and eye fasteners, snaps, clips, buttons, ties, and the like (not shown), for removably securing the ends together to hold the contact surface 22 against the skin 92. Alternatively, the subject or another person may simply hold the housing 20 and press the surface 22 against the skin 92 during use.

With continued reference to FIG. 1, the FUS transducer 30 may include a plurality of piezoelectric elements 31 providing an array such that the transducer 30 delivers focused ultrasound along a first axis 34, e.g., to generate a beam that focuses the ultrasonic energy at a desired location along the first axis 34, e.g., at a target nerve 94, as described further elsewhere herein. For example, as shown in FIG. 1, the transducer elements 31 may be mounted adjacent the contact surface 22 such that the elements 31 may be acoustically coupled to the skin 92 contacted by the surface 22 and ultrasonic energy generated by the elements 31 is transmitted along the first axis 34 from the surface 22 into the subject's body 90. In the example shown, the transducer elements 31 are mounted to or adjacent a substantially planar surface 32 in a substantially flat array, although it will be appreciated that the elements 31 may be provided in other arrangements, e.g., having a concave, convex or other arrangement centered on the axis 38 (not shown), as desired. In one example, the elements 31 may be positioned adjacent one another on the planar surface 32, e.g., in a circular arrangement on the surface 32 with one or more annular sets of elements 31, each including multiple elements 31 spaced apart radially relative to one another, or may be provided in a linear array, e.g., including one or more rows, each row including multiple square, rectangular, hexagonal, or otherwise shapes piezoelectric elements (not shown).

An acoustic pad 38 may be mounted or otherwise coupled to the planar surface 32 that defines the contact surface 22 of the device 10, e.g., to acoustically couple the transducer 30 to the subject's skin 92 and/or to facilitate pressing the device 10 against the skin 92. For example, the pad 38 may be a bag or other flexible membrane containing with acoustic gel, water, or other fluid, foam, or other material filling the space between the planar surface 32 and the contact surface 22 that enhances coupling the transducer 30 to the skin 92. Optionally, the pad 46 may be adjustable, e.g., along the first axis 34 to allow the focal zone of the ultrasonic energy to be manually adjusted relative to the subject's body 90 when pressed against the skin 92. For example, the membrane may be sufficiently flexible such that the distance between the surfaces 32 and 22 may be adjusted by displacing the gel or other pad material outwardly within the membrane, which may expand outwardly to allow displacement without rupturing.

Alternatively, the height between the surfaces 44, 2 may be adjusted using other mechanisms to adjust the depth of focus of the FUS energy. For example, in one alternative, a set of acoustic pads (not shown) may be provided having different dimensions, e.g., different heights, and/or different acoustic properties, and the user may select one for attachment to the device 10. In this alternative, the housing 20 may include one or more connectors adjacent the planar surface 32 that allow the selected pad to be mounted to housing 20 to provide the contact surface 22. The selected pad may be removed and replaced, as desired, e.g., if multiple locations are to be treated or if a single device is used to treat multiple subjects. This would allow a single device, i.e., with FUS transducer 30 and imaging element 40 to be customized by the user during or between uses. The pads may be sealed such that they may be sterilized or otherwise cleaned between uses or as desired.

With continued reference to FIG. 1, as shown, the imaging element 40 includes an imaging or diagnostic ultrasound transducer 42 configured to transmit incident ultrasound signals Dᵢ, e.g., from the contact surface 22 into the subject's body 90, e.g., centered on second axis 44, and receive reflected signals Dᵣ from the body 90. The imaging element 40 may be mounted to the housing 20 such that the imaging transducer 42 is offset from the FUS transducer 30, e.g., laterally relative to the planar surface 32, and angled such that the second axis 44 intersects the first axis 34. The imaging transducer 42 may include one or more piezoelectric elements (one shown for simplicity), e.g., provided at a distal end of an imaging housing 46 mounted adjacent the FUS transducer 30 such that the imaging signals Dᵢ, Dᵣ also pass through the pad 38. Alternatively, other imaging devices may be provided capable of generating signals that may be used to identify the target nerve 92 or other tissue of interest.

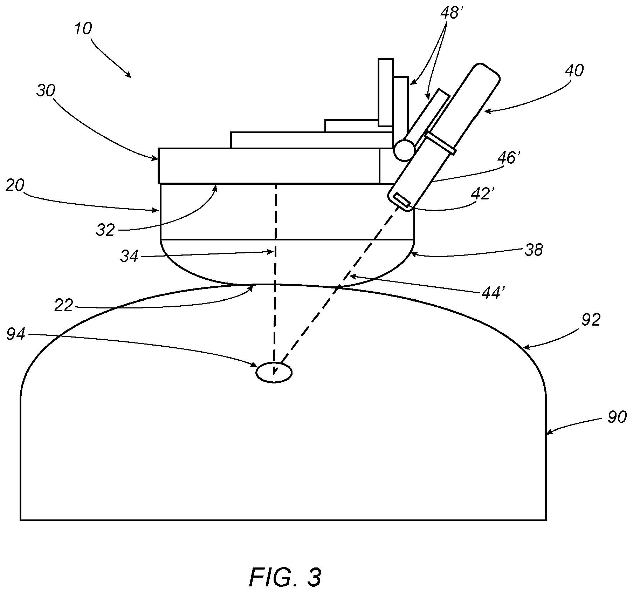

The imaging element 40 may be fixed relative to the housing 20, e.g., as shown in FIG. 1, such that the intersection angle of the axes 34, 44 is fixed (even if the angles may be otherwise modified electronically as described elsewhere herein). Alternatively, the imaging element 40 may be movable relative to the housing 20 to change the direction of the second axis 44. For example, as shown in FIG. 3, an imaging element 40' may be provided that is coupled to the housing 20 by a mechanism that provides one or more degrees of freedom of movement of the imaging element 40' relative to the housing 20. In the example shown, the imaging element 40' may be mounted to the housing 20 using a hinge 48' that allows the imaging element 40' to pivot relative to the housing 20 about a single axis, e.g., such that the imaging transducer 42 can be pivoted such that the second axis 44 may be substantially parallel to the first axis 34 and may intersect the first axis 34 at an increasing acute angle as the imaging element 40' is pivoted, e.g., between about zero and ninety degrees (0-90°). In addition or alternatively, the entire imaging element 40 may be movable spatially relative to the housing 20, e.g., in one or more directions within a plane parallel to the planar surface 32.

With additional reference to FIG. 2, a controller 50 (including one or more processors, memory, and/or other electronic components, not shown) is coupled to the imaging element 40 to control delivery of the incident signals Dᵢ and to process the reflected signals Dᵣ, e.g., to identify one or more tissue structures within the body 90. For example, the controller 50 may analyze the reflected signals to identify a target nerve 94 (or other tissue of interest) and determine the depth and/or other spatial information of the nerve 94 relative to the FUS transducer 30.

The controller 50 may also be coupled to the FUS transducer 30, e.g., to deliver energy to the transducer elements 31 to control delivery of the focused ultrasound to the target nerve 94 to relieve pain. The controller 50 may be coupled to the power source 60, e.g., including one or more batteries, transformers, and/or other components necessary to deliver signals to the transducer elements 31 to cause the elements 31 to generate acoustic energy directed along the first axis 34. In addition or alternatively, the device 10 may include a connector 62, e.g., to allow the device 10 to be connected to an external power source, e.g., simply plugged into an electrical outlet or an external power source capable of generating the power and/or signals necessary to operate the transducer 30 and/or imaging element 40. When activated, the device 10 may deliver the FUS energy substantially continuously, intermittently, and/or using a desired pulsative form, as desired. Optionally, the device 10 may include a user interface, e.g., touchscreen, set of buttons, and the like (not shown), allowing a user to select desired parameters of the energy delivered, e.g., to modify one or more of intensity, duration, waveform, and the like, if desired.

Optionally, the controller 50 may control one or more of phase, intensity, pulse duration, and frequency of the signals to the piezoelectric elements 31 to generate an ultrasound beam focused at a focal zone that may be directed on the target nerve 94. For example, based at least in part on the location of the target nerve 94 identified by the imaging element 40, the controller 50 may modify the signals to move the depth of the focal zone of the ultrasound beam B and/or to move the focal zone laterally relative to the first axis 34 to focus the acoustic energy on the target nerve 94. In addition, the controller 50 may identify different tissue structures between the skin 92 and the target nerve 94 based at least in part on signals from the imaging element 40, e.g., identifying skin thickness and/or the location and/or thickness of fat layers and muscle layers. The controller 50 may modify the signals to the elements 31 of the transducer 30 to take into account the differing ultrasound attenuation of the tissues along the path to the target nerve 92 to enhance focusing the acoustic energy. In addition or alternatively, if the imaging element 40 is movable, the orientation of the imaging element 40 may be manually adjusted and the controller 50 may modify the signals to adjust the focal zone based at least in part on the location of the target nerve

In addition or alternatively, the location of the focal zone may be manually adjusted, e.g., using the acoustic pad 38. For example, the distance from the planar surface 32 (and, consequently, the transducer elements 31) to the contact surface 22 may be adjusted by adjusting pressure applied to the pad 38, e.g., to displace gel or other material within the pad 38, to provide a desired distance to position the tissue of interest within the focal zone before delivering the FUS energy.

Alternatively, the FUS transducer 30 may be mounted within the housing 20 such that the FUS transducer 30 may be moved during use, e.g., using one or more servomotors or other actuators. For example, the housing 20 may be secured to the subject's body 90, e.g., using one or more straps or other features (not shown) and the controller 50 may process signals from the imaging element 40 to identify one or more regions within the body 90 for treatment. The transducer 30 may be activated continuously or intermittently and then moved within the housing 20 and, consequently, relative to the body 90 to move the focal zone. In one example, the transducer 30 may be movable within a plane parallel to the planar surface 32, thereby allowing the focal zone to be moved laterally relative to tissues within the body 90. For example, if the target treatment region is larger than the focal zone, the transducer 30 may be moved during a treatment do deliver acoustic energy to the entire region. Similarly, if the target region includes a long nerve, the transducer 30 may be moved to deliver FUS along a desired length of the nerve. Alternatively, the transducer 30 may be moved to deliver FUS to multiple regions with acoustic energy delivered to each region for a preset or customized duration.

Optionally, the device 10 may include an output device (not shown) that may be coupled to the controller 50 to assist a user in positioning the focal zone relative to the target nerve. For example, one or more light indicators, a speaker, and the like (not shown) may be provided on the housing 20 that may provide an output when the target nerve is within the focal zone. In addition or alternatively, a display (also not shown) may be provided on the housing 20 that may provide images based at least in part on the signals from the imaging element 40, e.g., identifying the location of the target nerve 94 (and/or other tissue structures) and superimposing a representation of the focal zone to allow the user to manually place the target nerve 94 within the focal zone. For example, the controller 50 may process signals from the imaging element 40 and present images on the display showing the location of the target nerve 94 relative to the focal zone. As the user moves the device 10, e.g., by pressing the pad 38 and/or moving the contact surface 22 along the skin 92, the location of the target nerve 94 may be monitored as it moves relative to the device 10 until the focal zone overlaps the target nerve 94 in the images, whereupon the user may activate the FUS transducer 30 to deliver the FUS energy, to relieve the subject's pain.

The device 10 may be configured to transmit low-intensity ultrasound, e.g., with an intensity up to five hundred Watts per square centimeter (i.e., between 0-500 W/cm²), e.g., at frequencies between about one kilohertz and ten megahertz (1 KHz-10 MHz). Alternatively, the device 10 may be configured to transmit high-intensity ultrasound, e.g., with an intensity between about five hundred and two thousand Watts per square centimeter (500-2000 W/cm²), e.g., at frequencies between about one kilohertz and ten megahertz (1 KHz-10 MHz). Thus, the device 10 may be used to temporarily relieve pain by applying FUS energy without damaging the target nerve or, alternatively, may be used to relieve pain indefinitely by causing neuromodulation, ablation, or other at least partial destruction of the nerve.

With additional reference to FIG. 1, during use, the device 10 may be used by a subject or a caregiver for relieving peripheral nerve pain in the subject. Generally, the contact surface 22 may be placed against the subject's skin 92, thereby acoustically coupling the FUS transducer 20 to the skin 92. Optionally, acoustic gel or other material may be applied to the skin 92 and/or to the contact surface 22 before placement to enhance acoustic coupling, if desired.

The imaging element 40 may be activated, whereupon the controller 50 may cause the imaging transducer 42 to transmit signals Dᵢ from the surface 22 into the subject's body 90 and receive reflected signals Dᵣ from the body 20 to identify a target nerve or other tissue of interest 94 within the body 90. Optionally, the device 10 may be moved along the skin 92 and/or the pad 38 may be pressed or released, as desired, e.g., under guidance from the imaging element 40. In addition or alternatively, if the imaging element 40 is movable, the device 10 may be applied to the skin 92 generally over the target nerve 94, and the imaging element 40 moved to identify the location relative to the FUS transducer 30, and the controller 50 may calibrate the signals necessary to direct the focal zone at the target nerve 94.

Once the target nerve 94 is within a focal zone of the FUS transducer 30, the FUS transducer 30 may be activated to deliver focused ultrasound to the target nerve 94, e.g., to relieve pain. Optionally, the FUS transducer 30 may be manually activated by the user, e.g., after confirming the location of the target nerve 94 (e.g., using an output device on the housing 20 to confirm). Alternatively, the controller 50 may automatically activate the FUS transducer 30 once it confirms that the target nerve 94 is within the focal zone. The FUS energy may be delivered for a preset time or the user may activate the FUS transducer 30 for a desired amount of time.

The devices, systems, and methods herein may be used to deliver focused ultrasound to a variety of peripheral verves to reduce acute and/or chronic pain, such as the lumbar plexus, femoral, saphenous, obturator, lateral femoral cutaneous, sciatic, posterior tibial, sural, common peroneal, deep peroneal, superficial peroneal, brachial plexus, intercostal brachial, musculocutaneous, median, radial, ulnar, ilioinguinal, iliohypogastric, intercostal, superficial cervical plexus, auriculotemporal, mental, buccal, infraorbital, supraorbital, supratrochlear, greater occipital, great auricular, and lesser occipital nerves. Potential conditions that may be treated include phantom limb pain, CRPS, type I & II, acute surgical pain: specific peripheral nerve blockade, neuroma, headaches, trigeminal neuralgia, Bell's palsy, glossopharyngeal nerve, intercostal pain, trigeminal neuralgia, neurolysis (e.g., celiac plexus, superior hypogastric plexus, etc.), endometriosis pain, shingles (e.g., post-herpatic neuralgia), rhizotomy, back pain, rheumatoid arthritis pain, cancer-related pain, and the like.

While the invention is susceptible to various modifications, and alternative forms, specific examples thereof have been shown in the drawings and are herein described in detail. It should be understood, however, that the invention is not to be limited to the particular forms or methods disclosed, but to the contrary, the invention is to cover all modifications and alternatives falling within the scope of the appended claims.

## Claims

1. A device (10) for relieving peripheral nerve pain in a subject, comprising:
a housing (20) including a surface (22) configured for placement against a subject's skin;
an imaging element (40) on the housing configured to transmit signals, Dᵢ, from the surface into the subject's body and receive reflected signals, Dᵣ, from the body;
one or more transducer elements (30) configured to deliver focused ultrasound, B, from the surface into the body; and
a controller (50) coupled to the imaging element to process the reflected signals to identify a target nerve within the body and coupled to the one or more transducer elements to control delivery of the focused ultrasound to the target nerve to relieve pain,
**characterized in that**:
the surface comprises a pad (38) configured to couple the one or more transducer elements to the skin;
the one or more transducer elements are configured to deliver the focused ultrasound along a first axis (34), and the imaging element is offset from the one or more transducers such that the imaging signals, Dᵢ, Dᵣ, are delivered along a second axis (44) that intersects the first axis.

2. The device of claim 1, wherein the one or more transducer elements comprise an array of piezoelectric elements (31) mounted adjacent the surface such that the array may be acoustically coupled to the skin.

3. The device of claim 2, wherein the pad comprises a flexible membrane containing foam.

4. The device of claim 2, wherein the controller is coupled to the array to control one or more of phase, intensity, pulse duration, and frequency of signals to the piezoelectric elements to generate an ultrasound beam focused on the target nerve.

5. The device of any one of claims 1-4, further comprising a power source (60) coupled to the one or more transducer elements and the controller to deliver electrical energy to the one or more transducer elements to deliver the focused ultrasound.

6. The device of claim 1, wherein the imaging element is movable relative to the housing to adjust an angle of the second axis relative to the first axis.

7. The device of claim 6, wherein the imaging element is mounted to the housing by a hinge (48') configured to adjust the angle of the second axis or wherein the device further comprises a handle mounted to the imaging element for adjusting the angle of the second axis.

8. The device of any one of claims 1-4, wherein the imaging element comprises an ultrasound imaging element (42, 42') configured to transmit ultrasound signals into the body and receive reflected ultrasound signals from tissue structures within the body and, optionally, wherein the ultrasound imaging element comprises an array of piezoelectric transducers.

9. The device of any one of claims 1-4, wherein the controller is configured to calibrate delivery of the focused ultrasound based at least in part on a distance from the one or more transducers to the target nerve.

10. The device of any one of claims 1-4, wherein the controller is configured to activate the one or more transducers to transmit low-intensity ultrasound at frequencies between about one kilohertz and ten megahertz and, optionally, wherein the intensity is between about zero and five hundred Watts per square centimeter .

11. The device of any one of claims 1-4, wherein the controller is configured to activate the one or more transducers to transmit high-intensity ultrasound at frequencies between about one kilohertz and ten megahertz and, optionally, wherein the intensity is between about five hundred and two thousand Watts per square centimeter

12. The device of any one of claims 1-4, wherein the controller is configured to activate the one or more transducers substantially continuously, intermittently, or in pulsatile form.

13. The device of any one of claims 1-4, wherein the controller is configured to activate the one or more transducers to deliver high-intensity ultrasound to cause thermal ablation or neurolysis of the target nerve for pain reduction or wherein the controller is configured to activate the one or more transducers to cause one or both excitation and suppression of nerve conduction.

14. The device of any preceding claim, wherein the housing is shaped to provide a handheld device, optionally, wherein the housing has an elongated cylindrical shape and one or more handles, grips, or other features extend from the housing to facilitate manipulation of the device during use.

15. The device of any preceding claim, further comprising an output device coupled to the controller to assist a user in positioning a focal zone of the one or more transducer elements relative to the target nerve, optionally, wherein the output device comprises one or more light indicators, a speaker, and the like provided on the housing that may provide an output when the target nerve is within the focal zone.

## Patentansprüche

1. Vorrichtung (10) zur Linderung von peripheren Nervenschmerzen in einem Subjekt, umfassend:
ein Gehäuse (20), das eine Oberfläche (22) einschließt, die so konfiguriert ist, dass sie gegen die Haut eines Subjekts platziert wird;
ein Bildgebungselement (40) auf dem Gehäuse, das so konfiguriert ist, dass es Signale, Dᵢ, von der Oberfläche in den Körper des Subjekts überträgt und reflektierte Signale, Dᵣ, vom Körper empfängt;
einen oder mehrere Wandlerelemente (30), die so konfiguriert sind, dass sie fokussierten Ultraschall, B, von der Oberfläche in den Körper abgeben; und
eine Steuereinheit (50), die mit dem Bildgebungselement gekoppelt ist zum Verarbeiten der reflektierten Signale, um einen Zielnerv innerhalb des Körpers zu identifizieren, und die mit dem einen oder den mehreren Wandlerelementen gekoppelt ist zum Steuern von Abgabe des fokussierten Ultraschalls an den Zielnerv, um Schmerzen zu lindern,
**dadurch gekennzeichnet, dass**:
die Oberfläche ein Pad (38) umfasst, das so konfiguriert ist, dass es das eine oder die mehreren Wandlerelemente mit der Haut koppelt;
wobei das eine oder die mehreren Wandlerelemente so konfiguriert sind, dass sie den fokussierten Ultraschall entlang einer ersten Achse (34) abgeben, und das Bildgebungselement von dem einen oder den mehreren Wandlern so versetzt ist, dass die Bildgebungssignale, Dᵢ, Dᵣ, entlang einer zweiten Achse (44), die die erste Achse schneidet, abgegeben werden.

2. Vorrichtung nach Anspruch 1, wobei das eine oder die mehreren Wandlerelemente eine Anordnung von piezoelektrischen Elementen (31) umfassen, die angrenzend zur Oberfläche so montiert sind, dass die Anordnung akustisch mit der Haut gekoppelt werden kann.

3. Vorrichtung nach Anspruch 2, wobei das Pad eine flexible Membran umfasst, die Schaumstoff enthält.

4. Vorrichtung nach Anspruch 2, wobei die Steuereinheit mit der Anordnung gekoppelt ist, um eines oder mehreres von Phasen, Intensität, Impulsdauer und Frequenz von Signalen an die piezoelektrischen Elemente zu steuern, um einen auf den Zielnerv fokussierten Ultraschallstrahl zu erzeugen.

5. Vorrichtung nach einem der Ansprüche 1-4, weiter umfassend eine mit dem einen oder den mehreren Wandlerelementen und der Steuereinheit gekoppelte Leistungsquelle (60) zum Abgeben von elektrischer Energie an das eine oder die mehreren Wandlerelemente zum Abgeben des fokussierten Ultraschalls.

6. Vorrichtung nach Anspruch 1, wobei das Bildgebungselement in Bezug auf das Gehäuse beweglich ist, um einen Winkel der zweiten Achse in Bezug auf die erste Achse anzupassen.

7. Vorrichtung nach Anspruch 6, wobei das Bildgebungselement durch ein Scharnier (48') am Gehäuse montiert ist, das so konfiguriert ist, dass es den Winkel der zweiten Achse anpasst, oder wobei die Vorrichtung weiter einen am Bildgebungselement montierten Griff zum Anpassen des Winkels der zweiten Achse umfasst.

8. Vorrichtung nach einem der Ansprüche 1-4, wobei das Bildgebungselement ein Ultraschall-Bildgebungselement (42, 42') umfasst, das so konfiguriert ist, dass es Ultraschallsignale in den Körper überträgt und reflektierte Ultraschallsignale von Gewebestrukturen innerhalb des Körpers empfängt, und optional wobei das Ultraschall-Bildgebungselement eine Anordnung von piezoelektrischen Wandlern umfasst.

9. Vorrichtung nach einem der Ansprüche 1-4, wobei die Steuereinheit so konfiguriert ist, dass sie Abgabe des fokussierten Ultraschalls zumindest teilweise basierend auf einem Abstand zwischen dem einen oder den mehreren Wandlern und dem Zielnerv kalibriert.

10. Vorrichtung nach einem der Ansprüche 1-4, wobei die Steuereinheit so konfiguriert ist, dass sie den einen oder die mehreren Wandler aktiviert, um Ultraschall niedriger Intensität mit Frequenzen zwischen etwa einem Kilohertz und zehn Megahertz zu übertragen, optional wobei die Intensität zwischen etwa null und fünfhundert Watt pro Quadratzentimeter liegt.

11. Vorrichtung nach einem der Ansprüche 1-4, wobei die Steuereinheit so konfiguriert ist, dass sie den einen oder die mehreren Wandler aktiviert, um hochintensiven Ultraschall mit Frequenzen zwischen etwa einem Kilohertz und zehn Megahertz zu übertragen, optional wobei die Intensität zwischen etwa fünfhundert und zweitausend Watt pro Quadratzentimeter liegt.

12. Vorrichtung nach einem der Ansprüche 1-4, wobei die Steuereinheit so konfiguriert ist, dass sie den einen oder die mehreren Wandler im Wesentlichen kontinuierlich, intermittierend oder in pulsierender Form aktiviert.

13. Vorrichtung nach einem der Ansprüche 1-4, wobei die Steuereinheit so konfiguriert ist, dass sie den einen oder die mehreren Wandler aktiviert, um hochintensiven Ultraschall abzugeben, um thermischen Ablation oder Neurolyse des Zielnervs zur Schmerzlinderung zu bewirken, oder wobei die Steuereinheit so konfiguriert ist, dass sie den einen oder die mehreren Wandler aktiviert, um eines oder beides von Erregung und Unterdrückung von Nervenleitung zu bewirken.

14. Vorrichtung nach einem vorstehenden Anspruch, wobei das Gehäuse so geformt ist, dass es eine tragbare Vorrichtung bereitstellt, optional wobei das Gehäuse eine längliche zylindrische Form aufweist und sich ein oder mehrere Griffe, Greifflächen oder andere Merkmale vom Gehäuse erstrecken, um Handhabung der Vorrichtung während des Gebrauchs zu erleichtern.

15. Vorrichtung nach einem vorstehenden Anspruch, weiter umfassend eine mit der Steuereinheit gekoppelte Ausgabevorrichtung, um einen Benutzer bei der Positionierung einer Fokuszone des einen oder der mehreren Wandlerelemente in Bezug auf den Zielnerv zu unterstützen, optional wobei die Ausgabevorrichtung eine oder mehrere auf dem Gehäuse bereitgestellte Lichtanzeigen, Lautsprecher und dergleichen umfasst, die eine Ausgabe bereitstellen können, wenn sich der Zielnerv innerhalb der Fokuszone befindet.

## Revendications

1. Dispositif (10) destiné à soulager la douleur des nerfs périphériques chez un sujet, comprenant :
un boîtier (20) incluant une surface (22) configurée pour être placée contre la peau d'un sujet ;
un élément d'imagerie (40) sur le boîtier configuré pour transmettre des signaux, Dᵢ, de la surface vers le corps du sujet et recevoir des signaux réfléchis, Dᵣ, du corps ;
un ou plusieurs éléments transducteurs (30) configurés pour délivrer des ultrasons focalisés, B, de la surface vers le corps ; et
un dispositif de commande (50) couplé à l'élément d'imagerie pour traiter les signaux réfléchis afin d'identifier un nerf cible dans le corps et couplé à un ou plusieurs éléments transducteurs pour commander la délivrance des ultrasons focalisés au nerf cible pour soulager la douleur,
**caractérisé en ce que** :
la surface comprend un coussinet (38) configuré pour coupler les un ou plusieurs éléments transducteurs à la peau ;
les un ou plusieurs éléments transducteurs sont configurés pour délivrer les ultrasons focalisés le long d'un premier axe (34), et l'élément d'imagerie est décalé par rapport aux un ou plusieurs transducteurs de sorte que les signaux d'imagerie, Dᵢ, Dᵣ, sont délivrés le long d'un second axe (44) qui croise le premier axe.

2. Dispositif selon la revendication 1, dans lequel les un ou plusieurs éléments transducteurs comprennent un réseau d'éléments piézoélectriques (31) monté de manière adjacente à la surface de sorte que le réseau puisse être couplé acoustiquement à la peau.

3. Dispositif selon la revendication 2, dans lequel le coussinet comprend une membrane flexible contenant de la mousse.

4. Dispositif selon la revendication 2, dans lequel le dispositif de commande couplé au réseau pour commander un ou plusieurs paramètres de phase, d'intensité, de durée d'impulsion et de fréquence des signaux envoyés aux éléments piézoélectriques pour générer un faisceau d'ultrasons focalisé sur le nerf cible.

5. Dispositif selon l'une quelconque des revendications 1-4, comprenant en outre une source d'énergie (60) couplée aux un ou plusieurs éléments transducteurs et au dispositif de commande pour fournir de l'énergie électrique aux un ou plusieurs éléments transducteurs afin de délivrer les ultrasons focalisés.

6. Dispositif selon la revendication 1, dans lequel l'élément d'imagerie est mobile par rapport au boîtier pour ajuster un angle du second axe par rapport au premier axe.

7. Dispositif selon la revendication 6, dans lequel l'élément d'imagerie est monté sur le boîtier par une charnière (48') configurée pour ajuster l'angle du second axe ou dans lequel le dispositif comprend en outre une poignée montée sur l'élément d'imagerie pour ajuster l'angle du second axe.

8. Dispositif selon l'une quelconque des revendications 1-4, dans lequel l'élément d'imagerie comprend un élément d'imagerie ultrasonore (42, 42') configuré pour transmettre des signaux ultrasonores dans le corps et recevoir des signaux ultrasonores réfléchis par les structures tissulaires à l'intérieur du corps et, en option, dans lequel l'élément d'imagerie ultrasonore comprend un réseau de transducteurs piézoélectriques.

9. Dispositif selon l'une quelconque des revendications 1-4, dans lequel le dispositif de commande est configuré pour calibrer la délivrance des ultrasons focalisés en fonction au moins partiellement de la distance entre les un ou plusieurs transducteurs et le nerf cible.

10. Dispositif selon l'une quelconque des revendications 1-4, dans lequel le dispositif de commande est configuré pour activer les un ou plusieurs transducteurs afin de transmettre des ultrasons de faible intensité à des fréquences comprises entre environ un kilohertz et dix mégahertz et, en option, dans lequel l'intensité est comprise entre environ zéro et cinq cents Watts par centimètre carré.

11. Dispositif selon l'une quelconque des revendications 1-4, dans lequel le dispositif de commande est configuré pour activer les un ou plusieurs transducteurs afin de transmettre des ultrasons de haute intensité à des fréquences comprises entre environ un kilohertz et dix mégahertz et, en option, dans lequel l'intensité est comprise entre environ cinq cents et deux mille Watts par centimètre carré.

12. Dispositif selon l'une quelconque des revendications 1-4, dans lequel le dispositif de commande est configuré pour activer les un ou plusieurs transducteurs de manière sensiblement continue, intermittente ou pulsatile.

13. Dispositif selon l'une quelconque des revendications 1-4, dans lequel le dispositif de commande est configuré pour activer les un ou plusieurs transducteurs afin de délivrer des ultrasons de haute intensité pour provoquer une ablation thermique ou une neurolyse du nerf cible pour une réduction de la douleur ou dans lequel le dispositif de commande est configuré pour activer les un ou plusieurs transducteurs afin de provoquer l'une ou les deux parmi l'excitation et la suppression de la conduction nerveuse.

14. Dispositif selon une quelconque revendication précédente, dans lequel le boîtier est conçu pour fournir un dispositif portatif, en option, dans lequel le boîtier présente une forme cylindrique allongée et une ou plusieurs poignées, zones de préhension ou autres caractéristiques s'étendent à partir du boîtier pour faciliter la manipulation du dispositif pendant l'utilisation.

15. Dispositif selon une quelconque revendication précédente, comprenant en outre un dispositif de sortie couplé au dispositif de commande pour aider un utilisateur à positionner une zone focale des un ou plusieurs éléments transducteurs par rapport au nerf cible, en option, dans lequel le dispositif de sortie comprend un ou plusieurs indicateurs lumineux, un haut-parleur, et analogues, disposés sur le boîtier et pouvant fournir une sortie lorsque le nerf cible se trouve dans la zone focale.
